# EUROPEAN PATENT SPECIFICATION

(11) **EP 3 160 481 B1**
(45) Date of publication and mention of the grant of the patent: **09.09.2020**
(21) Application number: 15742102.5
(22) Date of filing: 30.06.2015
(51) Int. Cl.: A61K 35/28, A61K 9/00, A61P 31/00

(54) **MESENCHYMAL STROMAL CELLS FOR TREATING SEPSIS**
MESENCHYMALE STROMAZELLEN ZUR BEHANDLUNG VON BLUTVERGIFTUNG
CELLULES STROMALES MESENCHYMATEUSES POUR LE TRAITEMENT DE LA SEPTICEMIE

(30) Priority: 30.06.2014 EP 14175095
(43) Date of publication of application: 03.05.2017
(73) Proprietor: TiGenix, S.A.U., 28760 Tres Cantos (Madrid) (ES)
(72) Inventor: DALEMANS, Wilfried, E-28760 Tres Cantos (Madrid) (ES); LOMBARDO, Eleuterio, E-28760 Tres Cantos (Madrid) (ES); DEKKER, Robert, E-28760 Tres Cantos (Madrid) (ES)
(74) Representative: Lasar, Andrea Gisela
(86) International application number: PCT/IB2015/054923
(87) International publication number: WO 2016/001846

(56) References cited:
- WO-A1-2005/093044
- WO-A2-2010/015929
- KRASNODEMBSKAYA ANNA ET AL: "Antibacterial Effect of Human Mesenchymal Stem Cells Is Mediated in Part from Secretion of the Antimicrobial Peptide LL-37", STEM CELLS (MIAMISBURG), vol. 28, no. 12, December 2010 (2010-12), pages 2229-2238, XP002744868, ISSN: 1066-5099
- KRISZTIÁN NÉMETH ET AL: "Bone marrow stromal cells attenuate sepsis via prostaglandin E2-dependent reprogramming of host macrophages to increase their interleukin-10 production", NATURE MEDICINE, vol. 15, no. 1, 1 January 2009 (2009-01-01), pages 42-49, XP055096324, ISSN: 1078-8956, DOI: 10.1038/nm.1905
- MEI SHIRLEY H J ET AL: "Mesenchymal Stem Cells Reduce Inflammation while Enhancing Bacterial Clearance and Improving Survival in Sepsis", AMERICAN JOURNAL OF RESPIRATORY AND CRITICAL CARE MEDICINE, vol. 182, no. 8, October 2010 (2010-10), pages 1047-1057, XP002744869,
- LI JIANJUN ET AL: "Human umbilical cord mesenchymal stem cells reduce systemic inflammation and attenuate LPS-induced acute lung injury in rats", JOURNAL OF INFLAMMATION-LONDON, vol. 9, September 2012 (2012-09), XP21121588, ISSN: 1476-9255

## Description

Systemic inflammatory response syndrome (SIRS) is an inflammatory state of the whole body without a specific source of infection. It can be caused by many factors, including but not limited to trauma, surgery, adrenal insufficiency, pulmonary embolism, myocardial infarction, hemorrhage, anaphylaxis, drug overdose, immunodeficiency and burns. There are four major diagnostic symptoms of SIRS, as listed below, but the presence of any two of these is sufficient for a diagnosis (see e.g. Nystrom (1998) Journal of Antimicrobial Chemotherapy, 41, Suppl. A, 1-7).
i) a heart rate in excess of 90 beats per minute;
ii) a body temperature below 36°C or above 38°C;
iii) a respiratory rate in excess of 20 breaths per minutes (tachypnea); and
vi) a white blood cell count below 4000 cells/mm³ or above 12000 cells/mm³, or the presence of more than 10% immature neutrophils.

SIRS causes widespread activation of acute phase immunogenic proteins, affecting the complement system and the coagulation pathways, which in turn cause damage to the vasculature as well as the internal organs. Various neuroendocrine counter-regulatory systems are subsequently activated, which often compound the problem.

Sepsis is a specific form of SIRS, and the most common cause of death in intensive care units. It is caused by a suspected or detected infection. It is characterized by a hyperactive and out-of-balance network of endogenous pro-inflammatory cytokines, and often leads to widespread inflammation and blood clotting, which may result in redness, heat, swelling, pain, organ dysfunction or organ failure. Blood clotting during sepsis may also cause reduced blood flow to the limbs and vital organs, and can lead to organ failure or the onset of gangrene.

Like SIRS, sepsis often results in an acute inflammation present throughout the body, and is therefore frequently associated with fever and leukocytosis (elevated white blood cell count). The current theory behind sepsis is that the host's immune response to the infection triggers SIRS, which in turn presents the symptoms described above. Following infection and sepsis, tissue perfusion and oxygen delivery may be reduced leading to septic shock. In order to be diagnosed with septic shock, there must be evidence of infection and refractory hypotension in the patient.

SIRS, sepsis and septic shock are severe medical conditions. Even with immediate and aggressive treatment, these diseases are likely to progress to multiple organ dysfunction syndrome and may even result in death.

Most therapeutic strategies to date have targeted pro-inflammatory mediators, but they have not been found to improve survival of patients when studied in large multi-center clinical trials. Therapies designed to block one single cytokine, such as TNFα and IL-1β, have shown limited efficacy probably due to the early and transient kinetic of these inflammatory cytokines. Recently, international critical care and infectious disease experts have developed management guidelines to improve the treatment given to patients suffering from SIRS, sepsis or septic shock. These guidelines aim to transform complex diagnostic and therapeutic decisions into simple "mission critical" tasks and, among other treatments, suggest the administration of broad-spectrum antibiotics, steroids and Drotrecogin Alfa (Activated).

However, mortality associated with sepsis remains at 30% to 50%, whilst the mortality rate for septic shock is reported to be even higher, at 50% to 60%. There are reported to be approximately 750,000 new sepsis cases each year, with at least 210,000 of these resulting in a fatality. As medical treatments become more aggressive, the incidences of SIRS, sepsis and septic shock are likely to increase, and consequently a new reliable treatment for these conditions is required.

WO 2010/015929 A2 relates to the use of mesenchymal stem cells for treating systemic inflammatory response syndrome.

### SUMMARY OF THE INVENTION

It has been found that administration of mesenchymal stromal cells (MSCs), in particular human adipose tissue derived stromal cells (hASCs), is useful in treating SIRS, sepsis, severe sepsis and septic shock. For example, MSCs, in particular hASCs, have been found to protect against mortality in pneumonia associated sepsis, providing evidence that MSCs may be useful in the treatment of SIRS, sepsis and septic shock. It has been found that MSCs function at several levels to regulate crucial aspects of SIRS, sepsis, severe sepsis and septic shock, including by reduction of systemic levels of various inflammatory cytokines and chemokines, and by inhibition of leukocyte infiltration into various target organs.

In one aspect, the invention therefore provides a composition comprising adipose tissue-derived stromal cells (MSCs), for use in treating sepsis in a human subject, wherein the sepsis is secondary to an inflammatory lung condition and wherein the composition is administered by an intravenous route.

The disclosure also provides the use of mesenchymal stromal cells (MSCs) in the manufacture of a medicament for treating sepsis subject having or subsequent to pneumonia.

The disclosure also provides a method of treating sepsis in a subject having or subsequent to pneumonia, comprising administering mesenchymal stromal cells (MSCs) to the subject.

These and other aspects of the invention are described in more detail below.

### BRIEF DESCRIPTION OF DRAWINGS

**Figures 1-4****:** Bacterial burden in various tissues at 48h post infection. A and B show the results from two independent experiments. PLACEBO = treatment with Ringer's lactate; ASC = treatment with ASC; CFU = colony forming units. Figure 1: Blood; Figure 2: Lung homogenate; Figure 3: Liver homogenate; and Figure 4: Spleen homogenate.
**Figures 5-8****:** Cytokine and chemokine levels in lung homogenates at 48h post infection. A and B show the results from two independent experiments. PLACEBO = treatment with Ringer's lactate; ASC = treatment with ASC. Figure 5: TNFα; Figure 6: IL1β; Figure 7: IL6; and Figure 8: Mip-2.
**Figures 9-12****:** Bacterial burden in various tissues post infection. PLACEBO = treatment with Ringer's lactate; ASC = treatment with ASC; CFU = colony forming units. Figure 9: Blood; Figure 10: Lung homogenate; Figure 11: Liver homogenate; and Figure 12: Spleen homogenate.

### DEFINITIONS

As used herein, the following terms and phrases shall have the meanings set forth below. Unless defined otherwise, all technical and scientific terms used herein have the same meaning as commonly understood to one of ordinary skill in the art to which this invention belongs.

The articles "a" and "an" refer to one or to more than one (i.e., to at least one) of the grammatical object of the article. By way of example, "an element" means one element or more than one element.

The term "about" when used in relation to a value relates to the value ±10%.

By "adipose tissue" is meant any fat tissue. The adipose tissue may be brown or white adipose tissue, derived from, for example, subcutaneous, omental/visceral, mammary, gonadal, or other adipose tissue site. Preferably, the adipose tissue is subcutaneous white adipose tissue. The adipose tissue may comprise a primary cell culture or an immortalized cell line. The adipose tissue may be from any organism having fat tissue. In some embodiments, the adipose tissue is mammalian, and in further embodiments the adipose tissue is human. A convenient source of adipose tissue is liposuction surgery. However, it will be understood that neither the source of adipose tissue nor the method of isolation of adipose tissue is critical to the invention. If cells as described herein are desired for autologous transplantation into a subject, the adipose tissue will be isolated from that subject.

"Adipose tissue-derived stromal cells (ASCs)" refers to MSCs that originate from adipose tissue, generally from human adipose tissue (hASCs).

The term "biological medicinal product" shall be taken to mean a protein or nucleic acid-based pharmaceutical substance for therapeutic use, which is typically produced by means other than direct extraction from a native (nonengineered) biological source.

The term "cells/kg" as used herein shall be taken to mean the number of cells (e.g. MSC) administered per kilogram of patient body weight.

The term "constitutively" is understood to mean the expression of a gene without any specific induction.

The term "culture" refers to the growth of cells, organisms, multicellular entities, or tissue in a medium. The term "culturing" refers to any method of achieving such growth, and may comprise multiple steps. The term "further culturing" refers to culturing a cell, organism, multicellular entity, or tissue to a certain stage of growth, then using another culturing method to bring said cell, organism, multicellular entity, or tissue to another stage of growth. A "cell culture" refers to a growth of cells in vitro. In such a culture, the cells proliferate, but they do not organize into tissue per se. A "tissue culture" refers to the maintenance or growth of tissue, e.g., explants of organ primordial or of an adult organ in vitro so as to preserve its architecture and function. A "monolayer culture" refers to a culture in which cells multiply in a suitable medium while mainly attached to each other and to a substrate. Furthermore, a "suspension culture" refers to a culture in which cells multiply while suspended in a suitable medium. Likewise, a "continuous flow culture" refers to the cultivation of cells or explants in a continuous flow of fresh medium to maintain cell growth, e.g. viability. The term "conditioned media" refers to the supernatant, e.g. free of the cultured cells/tissue, resulting after a period of time in contact with the cultured cells such that the media has been altered to include certain paracrine and/or autocrine factors produced by the cells and secreted into the culture. A "confluent culture" is a cell culture in which all the cells are in contact and thus the entire surface of the culture vessel is covered, and implies that the cells have also reached their maximum density, though confluence does not necessarily mean that division will cease or that the population will not increase in size.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. A "medium suitable for growth of a high density culture" is any medium that allows a cell culture to reach an OD600 of 3 or greater when other conditions (such as temperature and oxygen transfer rate) permit such growth. The term "basal medium" refers to a medium which promotes the growth of many types of microorganisms which do not require any special nutrient supplements. Most basal media generally comprise four basic chemical groups: amino acids, carbohydrates, inorganic salts, and vitamins. A basal medium generally serves as the basis for a more complex medium, to which supplements such as serum, buffers, growth factors, lipids, and the like are added. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM).

The terms "comprise" and "comprising" are used in the inclusive, open sense, meaning that additional elements may be included.

The term "expanded" as used herein when referring to cells shall be taken to have its usual meaning in the art, namely cells that have been proliferated in vitro. A MSC can be expanded to provide a population of cells that retain at least one biological function of the MSC, typically the ability to adhere to a plastic surface, under standard culture conditions. The expanded population of cells may retain the ability to differentiate into one or more cell types.

The term "including" is used herein to mean "including but not limited to". "Including" and "including but not limited to" are used interchangeably.

"Marker" refers to a biological molecule whose presence, concentration, activity, or phosphorylation state may be detected and used to identify the phenotype of a cell.

"Mesenchymal stromal cells (also referred to herein as "MSCs") are multipotent stromal cells, *i.e.* they are cells which are capable of giving rise to multiple different types of cells.

A "patient", "subject" or "host" to be treated by the subject method may mean either a human or non-human animal.

The term "pharmaceutical composition" refers to a composition intended for use in therapy. The compositions for use in the invention are pharmaceutical compositions, intended for use in treating sepsis, severe sepsis, septic shock and sepsis-like conditions. The compositions for use in the invention may include, in addition to MSCs, non-cellular components. Examples of such non-cellular components include but are not limited to cell culture media, which may comprise one or more of proteins, amino acids, nucleic acids, nucleotides, co-enzyme, anti-oxidants and metals.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient.

The term "phenotype" refers to the observable characteristics of a cell, such as size, morphology, protein expression, etc.

"Proliferation" refers to an increase in cell number. "Proliferating" and "proliferation" refer to cells undergoing mitosis.

"Refractory" shall be taken to mean having no significant clinical benefit when used in the treatment of a diseases e.g. no significant improvement or amelioration of symptoms or subsequent relapse of disease.

As used herein, the term "solution" includes a pharmaceutically acceptable carrier or diluent in which the MSCs used in the invention remain viable.

The term "substantially pure", with respect to MSC populations, refers to a population of cells in which at least about 75%, at least about 85%, at least about 90%, or at least about 95%, by number of the cells are MSCs. In other words, the term "substantially pure", with respect to MSC populations, refers to a population of cells that contains less than about 20%, less than about 10%, or less than about 5%, by number of lineage committed cells.

"Support" as used herein refers to any device or material that may serve as a foundation or matrix for the growth of adipose tissue-derived stromal stem cells.

"Therapeutic agent" or "therapeutic" refers to an agent capable of having a desired biological effect on a host. Chemotherapeutic and genotoxic agents are examples of therapeutic agents that are generally known to be chemical in origin, as opposed to biological, or cause a therapeutic effect by a particular mechanism of action, respectively. Examples of therapeutic agents of biological origin include growth factors, hormones, and cytokines. A variety of therapeutic agents are known in the art and may be identified by their effects. Certain therapeutic agents are capable of regulating cell proliferation and differentiation. Examples include chemotherapeutic nucleotides, drugs, hormones, non-specific (non-antibody) proteins, oligonucleotides (e.g., antisense oligonucleotides that bind to a target nucleic acid sequence (e.g., mRNA sequence)), peptides, and peptidomimetics.

### DETAILED DESCRIPTION

The invention provides a composition comprising adipose tissue-derived stromal cells (MSCs), for use in treating sepsis in a human subject, wherein the sepsis is secondary to an inflammatory lung condition and wherein the composition is administered by an intravenous route. The disclosure also provides the use of mesenchymal stromal cells (MSCs) in the manufacture of a medicament for treating sepsis in a subject. The disclosure also provides a method of treating sepsis in a subject comprising administering mesenchymal stromal cells (MSCs) to the subject. It will be understood that a "subject" in the context of this invention is typically a subject in need of treatment. Accordingly the disclosure also provides a method of treating sepsis in a subject in need thereof having or subsequent to pneumonia, comprising administering mesenchymal stromal cells (MSCs) to the subject.

Said sepsis is associated with, caused by or subsequent to an inflammatory lung condition, typically pneumonia. In one embodiment, the sepsis is secondary to pneumonia. Preferably said sepsis is severe sepsis and is associated with, caused by or subsequent to an inflammatory lung condition, typically pneumonia. In one embodiment, the sepsis is severe sepsis that is secondary to pneumonia. Said pneumonia may be caused by fungi, bacteria, or viruses. In one embodiment the pneumonia is caused by bacteria, for example gram-negative bacteria (gram-negative pneumonia). The gram-negative bacteria may comprise the *Klebsiella* genus, for example the species *Klebsiella pneumoniae,* such as *Klebsiella pneumoniae* serotype 2. In other embodiments the pneumonia is caused by *Streptococcus pneumonia,* in alternative embodiments the pneumonia may be caused by *Haemophilus influenzae, Chlamydophila pneumonia*, *Legionella pneumophila* or the fungus *Pneumocystis jiroveci.* The pneumonia may be community-acquired, healthcare-associated, hospital-acquired or ventilator-associated pneumonia. The pneumonia may be lobar pneumonia, bronchial pneumonia or acute interstitial pneumonia.

MSCs have been found to confer an immune-modulatory effect, particularly at sites of inflammation. It was found that exogenously administered MSCs typically settle in the lungs. Thus, MSCs are particularly effective in treating sepsis that is secondary to an inflammatory lung condition, such as pneumonia.

The examples show that intravenous administration of ASCs reduces the severity of pneumonia-associated sepsis, for example by reducing bacterial loads in various organs including blood and lungs, and reducing cytokine and chemokine levels in the lungs.

Accordingly, in one embodiment the invention provides ASCs for use in a method of treating pneumonia-associated sepsis in a human subject wherein administration by intravenous injection of MSCs reduces the systemic (*e.g*. blood) total bacterial load (CFU/ml) to statistically significant levels, for example by a factor of 10 or more, *e.g*. by a factor of 50 or more, or by a factor of 100 or more, compared to typical systemic total bacterial loads in untreated subjects. Total bacterial loads may be determined by methods well known to a person skilled in the art, e.g. semi-quantitative bacterial culture or quantitative PCR.

### CELLS FOR USE IN THE INVENTION

MSCs are undifferentiated stromal cells having the capacity to differentiate to other cells, and are typically derived from connective tissue, and are thus non-hematopoietic cells. The term "connective tissue" refers to tissue derived from mesenchyme and includes several tissues which are characterized in that their cells are included within the extracellular matrix. Among the different types of connective tissues, adipose and cartilaginous tissues are included. In one embodiment, the MSCs are from the stromal fraction of the adipose tissue. Alternative sources of MSCs include, but are not limited to, chondrocytes, e.g. from hyaline cartilage, skin, bone marrow, periosteum, dental pulp, spleen, pancreas, ligament, tendon, skeletal muscle, umbilical cord and placenta.

The MSCs can be obtained from any suitable source and from any suitable animal, including humans. Typically, said cells are obtained from post-natal mammalian connective tissues. The MSCs are obtained from the stromal fraction of adipose tissue. Also, in a particular embodiment, the MSCs are from a mammal, e.g., a rodent, primate, etc., preferably, from a human. Typically, the MSCs are obtained from the stromal fraction of human adipose tissue, i.e. they are adipose tissue-derived stromal cells (ASCs).

The MSCs are adherent to plastic under standard culture conditions.

MSCs are undifferentiated multipotent cells, having the capacity to differentiate into or towards somatic cells such as mesodermal cells (e.g. adipose, chondrocytes, osteoblasts) and optionally into or towards endodermal and/or ectodermal cell types or lineages. Typically the cells have the capacity to differentiate into or towards at least two or all cell types selected from the group consisting of adipocytic, chondroblastic and osteoblastic lineages.

The MSCs are adipose tissue derived stem cells. Typically the MSCs (i) do not express markers specific from APCs; (ii) do not express IDO constitutively (iii) do not significantly express MHC II constitutively. Typically expression of IDO or MHC II may be induced by stimulation with IFN-γ.

Typically the MSCs may express one or more (*e.g*. two or more, three or more, four or more, five or more, six or more, seven or more, eight or more, nine or more, or ten or more *(e.g.* up to 13)) of the markers CD9, CD10, CD13, CD29, CD44, CD49A, CD51, CD54, CD55, CD58, CD59, CD90 and CD105. For example, the MSCs may express one or more (*e.g*. two, three or all) of the markers CD29, CD59, CD90 and CD105, e.g. CD59 and/or CD90.

Typically the MSCs may not express one or more (*e.*g. two or more, three or more, four or more, five or more, six or more, seven or more, eight or more, nine or more, or ten or more (*e.g.* up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs do not express one or more (e.g. two, three or all) of the markers CD34, CD45, CD31 and CD14, e.g. CD31 and/or CD34.

### EXPANDED MSCs

In one embodiment the MSCs are *in vitro* culture expanded MSCs or the *in vitro* culture expanded progeny thereof (hereinafter both are referred to as expanded MSCs or "eMSC"). Methods for the preparation of eMSCs are known in the art, for example as described in WO2007039150. eMSCs retain several phenotypic characteristics of MSCs, e.g. the eMSCs are adherent to plastic under standard culture conditions and retain an undifferentiated phenotype.

eMSCs are undifferentiated multipotent cells, having the capacity to differentiate into somatic cells such as mesodermal cells. Whereas MSCs have the capacity to differentiate towards at least one or more specialized cell lineages such as but not limited to adipocytic, chondroblastic and osteoblastic lineages; typically in eMSCs this capacity to differentiate is reduced or may even be absent e.g. whereas a MSCs may differentiate towards at least the osteogenic and adipocytic lineages, the eMSCs derived therefrom may differentiate only towards the adipocytic lineage. This may be advantageous for therapeutic applications of the cells, where the cells may be administered to patients as it can reasonably be expected that unanticipated and potentially harmful differentiation of eMSCs will be less likely to occur.

In one embodiment the eMSCs may be the progeny of stem cells. Typically the eMSCs (i) do not express markers specific from APCs; (ii) do not express IDO constitutively (iii) do not significantly express MHC II constitutively. Typically expression of IDO or MHC II may be induced by stimulation with IFN-γ.

Typically the eMSCs may express one or more (*e.g*. two or more, three or more, four or more, five or more, six or more, seven or more, eight or more, nine or more, or ten or more *(e.g.* up to 13)) of the markers CD9, CD10, CD13, CD29, CD44, CD49A, CD51, CD54, CD55, CD58, CD59, CD90 and CD105, e.g. the MSCs may express one or more (*e.g*. two, three or all) of the markers CD29, CD59, CD90 and CD105, e.g. CD59 and/or CD90.

Typically the eMSCs may not express one or more (*e.g*. two or more, three or more, four or more, five or more, six or more, seven or more, eight or more, nine or more, or ten or more (*e.g.* up to 15)) of the markers Factor VIII, alpha-actin, desmin, S-100, keratin ,CD11b, CD11c, CD14, CD45, HLAII, CD31, CD34, CD45, STRO-1 and CD133, e.g. the MSCs do not express one or more (e.g. two, three or all) of the markers CD34, CD45, CD31 and CD14, e.g. CD31 and/or CD34. Furthermore the MSCs may optionally not express the marker STRO-1.

### CELL POPULATIONS

In one aspect the present invention provides populations of MSCs and/or eMSCs for therapeutic uses as described herein, these populations may hereinafter be referred to as "cell populations for use in the invention". Typically, the MSCs are expanded human ASCs, typically allogeneic expanded human ASCs. Typically the cell populations for use in the invention are a homogenous or substantially homogenous population of MSCs and/or eMSCs. Cell populations for use in the invention comprise or comprise essentially of MSCs and/or eMSCs, however cell populations of the invention may also comprise other cell types. Accordingly in one embodiment the invention provides cell populations for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are MSCs and/or eMSCs.

Typically the cell populations for use in the invention are a culture expanded population of MSCs, comprising or comprising essentially of eMSCs, however cell populations for use in the invention may also comprise other cell types. Accordingly in one embodiment the invention provides cell populations for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells are eMSCs. The eMSCs are eASCs, for example human eASCs. In a particular embodiment, the cells are allogeneic with respect to the subject to be treated.

Typically a cell population for use in the invention may have the capacity to differentiate towards at least one or more specialized cell lineages such as but not limited to adipocytic, chondroblastic and osteoblastic lineages. In one embodiment a cell population for use in the invention may have the capacity to differentiate into or towards at least two or all cell types selected from the group consisting of adipocytic, chondroblastic and osteoblastic lineages. However in an alternative embodiment this capacity to differentiate is reduced or may even be absent e.g. whereas a MSC may differentiate towards at least the osteogenic and adipocytic lineages, the eMSC population derived therefrom may differentiate only towards the adipocytic lineage. This may be advantageous for therapeutic applications of the cells, where the cells may be administered to patients as it can reasonably be expected that unanticipated and potentially harmful differentiation of eMSCs will be less likely to occur.

Typically a cell population for use in the invention may express one or more (*e.g*. two or more, three or more, four or more, five or more, six or more, seven or more, eight or more, nine or more, or ten or more (*e.g.* up to 13)) of the markers CD9, CD10, CD13, CD29, CD44, CD49A, CD51, CD54, CD55, CD58, CD59, CD90 and CD105, e.g. the MSCs may express one or more (*e.g*. two, three or all) of the markers CD29, CD59, CD90 and CD105, e.g. CD59 and/or CD90. Accordingly in one embodiment the invention provides a cell population for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells express one or more *(e.g.* two or more, three or more, four or more, five or more, six or more, seven or more, eight or more, nine or more, or ten or more *(e.g.* up to 13)) of the markers CD9, CD10, CD13, CD29, CD44, CD49A, CD51, CD54, CD55, CD58, CD59, CD90 and CD105, e.g. the MSCs may express one or more *(e.g.* two, three or all) of the markers CD29, CD59, CD90 and CD105, e.g. CD59 and/or CD90. In an alternative embodiment the invention provides cell populations for use in the invention in which the level of expression of one or more (*e.g*. two or more, three or more, four or more, five or more, six or more, seven or more, eight or more, nine or more, or ten or more *(e.g.* up to 13)) of the markers CD9, CD10, CD13, CD29, CD44, CD49A, CD51, CD54, CD55, CD58, CD59, CD90 and CD105 is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, e.g. a cell population for use in the invention may express one or more (*e.g*. two, three or all) of the markers CD29, CD59, CD90 and CD105, e.g. CD59 and/or CD90 at the afore-mentioned level.

Typically a cell population for use in the invention may not express one or more (*e.g.* two or more, three or more, four or more, five or more, six or more, seven or more, eight or more, nine or more, or ten or more (*e.g.* up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs do not express one or more (*e.g*. two, three or all) of the markers CD34, CD45; CD31; CD14 e.g. CD31 and/or CD34. Furthermore the MSCs may optionally not express the marker STRO-1.

Accordingly in one embodiment the invention provides a cell population for use in the invention in which at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%, of the cells do not express one or more (*e.g*. two or more, three or more, four or more, five or more, six or more, seven or more, eight or more, nine or more, or ten or more (*e.g.* up to 15)) of the markers Factor VIII, alpha-actin, desmin, S-100, keratin, CD11b, CD11c, CD14, CD45, HLAII, CD31, CD34, CD45, STRO-1 and CD133, e.g. the MSCs may express one or more (*e.g*. two, three or all) of the markers CD34, CD45, CD31 and CD14, e.g. CD31 and/or CD34. In an alternative embodiment the invention provides cell populations for use in the invention in which the level of expression of one or more (e.g. two or more, three or more, four or more, five or more, six or more, seven or more, eight or more, nine or more, or ten or more (*e.g.* up to 15)) of the markers Factor VIII, alpha-actin, desmin, S-100, keratin, CD11b, CD11c, CD14, CD45, HLAII, CD31, CD34, CD45, STRO-1 and CD133 is below at least about 35%, at least about 30%, at least about 25%, at least about 20%, at least about 25%, at least about 5%, at least about 1%, e.g. the cell populations for use in the invention may express one or more (*e.g*. two, three or all) of the markers CD34, CD45, CD31 and CD14, e.g. CD31 and/or CD34 at the afore-mentioned level.

In some embodiments MSCs or eMSCs are pre-stimulated in order to enhance one or more of their proliferation capacity, migration capacity, survival capacity, therapeutic effect and inmunoregulatory properties. Typically, at least about 40% (e.g. at least about 50%, at least about 60%, at least about 70%, at least about 80%, at least about 85%, at least about 90%, at least about 95% at least about 96%, at least about 97%, at least about 98%, or at least about 99%) of the cell populations for use in the invention are pre-stimulated in order to enhance one or more of their proliferation capacity, migration capacity, survival capacity, therapeutic effect and inmunoregulatory properties. In some embodiments, pre-stimulation may be achieved by contacting the MSCs with a cytokine. In some embodiments of the invention, pre-stimulation may be achieved by contacting the MSCs with IFN-γ.

### COMPOSITIONS OF THE INVENTION

In one embodiment the present invention provides compositions comprising cell populations for use in the invention for use in methods of treatment according to the present invention. It is preferred that said composition is a pharmaceutical composition. A composition for use in the invention may include a substantially pure population of ASCs or eASCs, e.g. human eASCs. The MSCs, eMSCs, ASCs or eASCs may be stem cells. The composition of the present invention may also include cell culture components, e.g., culture media including one or more of amino acids, metals and coenzyme factors. The composition may include small populations of other stromal cells. The composition may also include other non-cellular components which may support the growth and survival of the MSCs under particular circumstances, *e.g*. implantation, growth in continuous culture, or use as a biomaterial or composition.

The concentration of the MSCs and/or eMSCs in the composition for use in the invention may be at least about 1 x 10⁴ cells/mL, at least about 1 x 10⁵ cells/mL, at least about 1 x 10⁶ cells/mL, at least about 10 x 10⁶ cells/mL, or at least about 40 x 10⁶ cells/mL. Typically the concentration between about 1 x 10⁶ cells/mL and 1 x 10⁷ cells/mL, e.g. between about between about 5 x 10⁶ cells/mL and 1 x 10⁷ cells/mL.

The compositions for use in the invention will generally comprise a pharmaceutically acceptable carrier and/or a diluent. Examples of such carriers and diluents are well known in the art, and may include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations. Typically the compositions for use in the invention comprise DMEM as the carrier, which may optionally be supplemented with serum (e.g. HSA) at a concentration of e.g. up to about 5% , up to about 10%, up to about 15%, up to about 20%, up to about 25% , up to about 30% .

A composition for use in the invention may be sterile and/or fluid to the extent that easy syringability exists. In addition, the composition may be stable under the conditions of manufacture and storage and preserved against the contaminating action of microorganisms such as bacteria and fungi through the use of, for example, parabens, chlorobutanol, phenol, ascorbic acid and thimerosal.

### PREPARATION METHODS

Methods for the isolation and culture of MSCs to provide eMSCs and cell populations for use in the invention and compositions comprising cell populations for use in the invention are known in the art. Typically methods for the preparation of compositions comprising cell populations comprise the following steps:
(i) isolation of MSCs from tissue and selection by adherence to a suitable surface e.g. plastic
(ii) expansion of MSCs to provide cell populations comprising eMSCs.

Optionally the cell populations for use in the invention may be cryopreserved during and/or subsequent to the expansion step (ii). Optionally the phenotype of the cell populations for use in the invention may be assessed during and/or subsequent to the expansion step (ii). Optionally the cell populations for use in the invention may be isolated subsequent to the expansion step (ii) and resuspended in a pharmaceutically acceptable carrier and/or diluents.

MSCs may be isolated from any suitable tissue, such as but not limited to peripheral blood, bone marrow, placenta, adipose tissue, periosteum, dental pulp, spleen, pancreas, ligament, skin, tendon, skeletal muscle, umbilical cord and placenta. The MSCs are obtained from the stromal fraction of adipose tissue.

The MSCs can be obtained from any suitable source and from any suitable animal, including humans. Typically, said cells are obtained from post-natal mammalian connective tissues.

The MSCs may also be isolated from any organism of the same or different species as the subject. Any organism with MSCs can be a potential candidate. In one embodiment the organism may be mammalian, and in another embodiment the organism is human.

Adipose-derived MSCs can be obtained by any means standard in the art. Typically said cells are obtained by disassociating the cells from the source tissue (e.g. lipoaspirate or adipose tissue), typically by treating the tissue with a digestive enzyme such as collagenase. The digested tissue matter is then typically filtered through a filter of between about 20 microns to 1mm. The cells are then isolated (typically by centrifugation) and cultured on an adherent surface (typically tissue culture plates or flasks). Such methods are known in the art and e.g. as disclosed in U.S. Patent No. 6777231. According to this methodology, lipoaspirates are obtained from adipose tissue and the cells derived therefrom. In the course of this methodology, the cells may be washed to remove contaminating debris and red blood cells, preferably with PBS. The cells are digested with collagenase (e.g. at 37°C for 30 minutes, 0.075% collagenase; Type I, Invitrogen, Carlsbad, CA) in PBS. To eliminate remaining red blood cells, the digested sample can be washed (e.g. with 10% fetal bovine serum), treated with 160 mmol/L ClNH4, and finally suspended in DMEM complete medium (DMEM containing 10% FBS, 2 mmol/L glutamine and 1% penicillin/streptomycin). The cells can be filtered through a 40-µm nylon mesh.

The cells are cultured in a suitable tissue culture vessel, comprising a surface suitable for the adherence of MSCs e.g. plastic. Non-adherent cells are removed e.g. by washing in a suitable buffer, to provide an isolated population of adherent stromal cells (e.g. MSCs). Cells isolated in this way can be seeded (preferably 2-3x10⁴ cells/cm²) onto tissue culture flasks and expanded at 37°C and 5% CO₂, changing the culture medium every 3-4 days. Cells are preferably passed to a new culture flask (1,000 cells/cm2) when cultures reach 90% of confluence.

Cell isolation is preferably carried out under sterile or GMP conditions.

In certain embodiments, the cells may be cultured for at least about 15 days, at least about 20 days, at least about 25 days, or at least about 30 days. Typically the expansion of cells in culture improves the homogeneity of the cell phenotype in the cell population, such that a substantially pure or homogenous population is obtained.

Cells are preferably detached from the adherent surface (e.g. by means of trypsin) and transferred to a new culture vessel (passaged) when cultures reach about 75%, 80%, 85%, 90% or 95% confluence.

In certain embodiments, the cells are multiplied in culture for at least three culture passages or "passaged at least three times". In other embodiments, the cells are passaged at least four times, at least five times, at least six times, at least seven times, at least eight times, at least nine times, or at least ten times.

In certain embodiments, the cells are expanded in culture for at least three population doublings. In certain embodiments, the cells are expanded in culture for at least four, five, six, seven, eight, nine, ten or 15 population doublings. In certain embodiments, the cells are expanded in culture for less than seven, eight, nine, ten or 15 population doublings. In certain embodiments, the cells are expanded in culture for between about 5 and 10 population doublings.

Cells may be cultured by any technique known in the art for the culturing of stromal stem cells. A discussion of various culture techniques, as well as their scale-up, may be found in Freshney, R.I., Culture of Animal Cells: A Manual of Basic Technique, 4th Edition, Wiley-Liss 2000. Cells may be expanded using culture flasks or bioreactors suitable for large-scale expansion. Bioreactors suitable for the large-scale expansion of mesenchymal stromal cells are commercially available and may include both 2D (i.e. substantially planar) and 3D expansion bioreactors. Examples of such bioreactors include, but are not limited to, a plug flow bioreactor, a perfusion bioreactor, a continuous stirred tank bioreactor, a stationary-bed bioreactor.

In certain embodiments, the cells are cultured by monolayer culture. Any medium capable of supporting MSCs in tissue culture may be used. Media formulations that will support the growth of MSCs include, but are not limited to, Dulbecco's Modified Eagle's Medium (DMEM), alpha modified Minimal Essential Medium (αMEM), and Roswell Park Memorial Institute Media 1640 (RPMI Media 1640). Typically, 0 to 20% Fetal Bovine Serum (FBS) will be added to the above media in order to support the growth of stromal cells. However, a defined medium could be used if the necessary growth factors, cytokines, and hormones in FBS for stromal cells and chondrocytes are identified and provided at appropriate concentrations in the growth medium. Media useful may contain one or more compounds of interest, including, but not limited to antibiotics, mitogenic or differentiating compounds for stromal cells. The cells for use in the invention may be grown at temperatures between 31°C to 37°C in a humidified incubator. The carbon dioxide content may be maintained between 2% to 10% and the oxygen content may be maintained at between 1% and 22%.

Antibiotics which can be added to the medium include, but are not limited to penicillin and streptomycin. The concentration of penicillin in the chemically defined culture medium may be about 10 to about 200 units per ml. The concentration of streptomycin in the chemically defined culture medium may be about 10 to about 200 ug/ml.

Typically, the MSCs as used in the present invention are expanded cell populations, preferably said cells are expanded to provide a substantially pure or homogenous population.

In one embodiment said cell populations are expanded until expression of the marker CD34 is reduced compared to freshly isolated, non-expanded cells. For example the cell population is expanded until expression of the marker CD34 is reduced to a level of less than 50%, less than 35%, less than 30%, or less than 5%, e.g. 35-5%, or 20-10%, of cells in the population. Typically, the cell population is expanded until expression of the marker CD34 is reduced to a level of less 5% of cells in the population. Thus, a population of eMSCs for use in the invention comprises less than 50%, less than 35%, less than 30%, or less than 5%, e.g. 35-5%, or 20-10%, of cells expressing CD34.

In one embodiment said cell populations are expanded until expression of the marker STRO-1 is reduced compared to freshly isolated, non-expanded cells. For example the cell population is expanded until expression of the marker STRO-1 is reduced to a level of less than 50%, less than 35%, less than 30%, or less than 5%, e.g. 35-5%, or 20-10%, of cells in the population. Typically, the cell population is expanded until expression of the marker STRO-1 is reduced to a level of less 5% of cells in the population. Thus, a population of eMSCs for use in the invention comprises less than 50%, less than 35%, less than 30%, or less than 5%, e.g. 35-5%, or 20-10%, of cells expressing STRO-1.

Typically, the cell populations are expanded until expression of the markers CD34 and STRO-1 is reduced compared to freshly isolated, non-expanded cells. For example the cell population is expanded until expression of the markers CD34 and STRO-1 is reduced to a level of less than 50%, less than 35%, less than 30%, or less than 5%, e.g. 35-5%, or 20-10%, of cells in the population. Typically, the cell population is expanded until expression of the markers CD34 and STRO-1 is reduced to a level of less 5% of cells in the population. Thus, a population of eMSCs for use in the invention comprises less than 50%, less than 35%, less than 30%, or less than 5%, e.g. 35-5%, or 20-10%, of cells expressing CD34 and STRO-1.

Expanded MSC populations (e.g. those expressing 5% or less CD34 and/or STRO-1) are advantageous as they present a lower multipotency than freshly isolated cells, i.e. they may have a lower, reduced or no capacity to differentiate into other cell phenotypes as compared to naturally-occurring non-expanded MSCs.

It will be apparent to one skilled in the art that the method for preparation of the composition is not limiting, and that compositions prepared in any way are included. The disclosure provides a method of preparing a composition, which comprises: (a) collecting tissue from a donor; (b) obtaining a cell suspension by enzymatic treatment; (c) sedimenting the cell suspension and re-suspending the cells in a culture medium; (d) culturing the cells for at least about 5 days or 5 population doublings.

In one embodiment the cells may be cryopreserved prior to administration, e.g. during and/or subsequent to expansion. Thus, the disclosure also provides cryopreserved cells. Methods for cell cryopreservation are known in the art, and typically require the use of suitable cryoprotective agents (e.g. DMSO). Cells may be cryopreserved at any point during the isolation and/or expansion stages and thawed prior to administration. Typically cells may be cryopreserved at passage 3, 4, 5, 6, 7, 8, 9, 10, 12, 15 or higher, e.g. cells may be cryopreserved at passages 3-10 or 5-10. Optionally the cells may be replated and cultured prior to administration.

In one embodiment the cells may be isolated from the cryopreservation and/or culture media and resuspended in a pharmaceutically acceptable carrier and/or diluents prior to administration (e.g. DMEM, optionally supplemented with serum).

In some embodiments, the cell populations for use in a composition of the invention may be pre-stimulated in order to enhance one or more of their proliferation capacity, migration capacity, survival capacity, therapeutic effect and inmunoregulatory properties. In some embodiments, pre-stimulation may be achieved by contacting the MSCs with a cytokine. In some embodiments of the invention, pre-stimulation may be achieved by contacting the MSCs with IFN-γ.

In certain embodiments of the invention, the MSCs may be pre-stimulated using a concentration of IFN-γ between 0.1 and 100 ng/ml. In further embodiments, the MSCs may be pre-stimulated using a concentration of IFN-γ between 0.5 and 85 ng/ml, between 1 and 70 ng/ml, between 1.5 and 50 ng/ml, between 2.5 and 40 ng/ml, or between 3 and 30 ng/ml. Pre-stimulation may occur over a stimulation time longer than about 12 hours. Pre-stimulation may occur over a stimulation time longer than about 13 hours, longer than about 18 hours, longer than about 24 hours, longer than about 48 hours, or longer than about 72 hours.

In one embodiment, the MSCs for use in the invention may be stably or transiently transfected or transduced with a nucleic acid of interest using a plasmid, viral or alternative vector strategy. Nucleic acids of interest include, but are not limited to, those encoding gene products which enhance the production of extracellular matrix components found in the tissue type to be repaired, e.g. intestinal wall or vaginal wall.

The transduction of viral vectors carrying regulatory genes into the stromal cells can be performed with viral vectors, including but not limited to adenovirus, retrovirus or adeno-associated virus purified (e.g. by cesium chloride banding) at a multiplicity of infection (viral units:cell) of between about 10:1 to 2000:1. Cells may be exposed to the virus in serum free or serum-containing medium in the absence or presence of a cationic detergent such as polyethyleneimine or Lipofectamine™ for a period of about 1 hour to about 24 hours (Byk T. et al. (1998) Human Gene Therapy 9:2493-2502; Sommer B. et al. (1999) Calcif. Tissue Int. 64:45-49).

Other suitable methods for transferring vectors or plasmids into stromal cells include lipid/DNA complexes, such as those described in U.S. Pat. Nos. 5,578,475; 5,627,175; 5,705,308; 5,744,335; 5,976,567; 6,020,202; and 6,051,429. Suitable reagents include lipofectamine, a 3:1 (w/w) liposome formulation of the poly-cationic lipid 2,3-dioleyloxy-N-[2(sperminecarbox- amido)ethyl]-N,N-dimethyl-1-propanaminium trifluoroacetate (DOSPA) (Chemical Abstracts Registry name: N-[2-(2,5-bis[(3-aminopropyl)amino]-1--oxpentyl}amino) ethyl]-N,N-dimethyl-2,3-bis(9-octadecenyloxy)-1-propanamin- ium trifluoroacetate), and the neutral lipid dioleoyl phosphatidylethanolamine (DOPE) in membrane filtered water. Exemplary is the formulation Lipofectamine 2000TM (available from Gibco/Life Technologies # 11668019). Other reagents include: FuGENETM 6 Transfection Reagent (a blend of lipids in non-liposomal form and other compounds in 80% ethanol, obtainable from Roche Diagnostics Corp. # 1814443); and LipoTAXITM transfection reagent (a lipid formulation from Invitrogen Corp., #204110). Transfection of stromal cells can be performed by electroporation, e.g., as described in M.L. Roach and J.D. McNeish (2002) Methods in Mol. Biol. 185:1. Suitable viral vector systems for producing stromal cells with stable genetic alterations may be based on adenoviruses and retroviruses, and may be prepared using commercially available virus components.

The transfection of plasmid vectors carrying regulatory genes into the MSCs can be achieved in monolayer cultures by the use of calcium phosphate DNA precipitation or cationic detergent methods (Lipofectamine™, DOTAP) or in three dimensional cultures by the incorporation of the plasmid DNA vectors directly into the biocompatible polymer (Bonadio J. et al. (1999) Nat. Med. 5:753-759).

For the tracking and detection of functional proteins encoded by these genes, the viral or plasmid DNA vectors may contain a readily detectable marker gene, such as the green fluorescent protein or beta-galactosidase enzyme, both of which can be tracked by histochemical means.

Subsequent to expansion it is preferred that cell populations for use in the invention are assayed to determine the expression of characteristic markers to confirm their phenotype, which can be carried out by using conventional means.

The term "expressed" is used to describe the presence of a marker within a cell. In order to be considered as being expressed, a marker must be present at a detectable level. By "detectable level" is meant that the marker can be detected using one of the standard laboratory methodologies such as PCR, blotting or FACS analysis. The phenotypic surface marker characterization of a population of MSCs may be performed by any method known in the art. By way of example, but not limitation, this phenotypic characterization may be performed by individual cell staining. Such staining may be achieved through the use of antibodies. This may be direct staining, by using a labeled antibody or indirect staining, using a second labeled antibody against a primary antibody specific for the cell marker. Antibody binding may be detected by any method known in the art. Antibody binding may also be detected by flow cytometry, immunofluorescence microscopy or radiography.

Alternatively or additionally, a gene is considered to be expressed by a cell of the population for use in the invention if expression can be reasonably detected after 30 PCR cycles, which corresponds to an expression level in the cell of at least about 100 copies per cell. The terms "express" and "expression" have corresponding meanings. At an expression level below this threshold, a marker is considered not to be expressed. The comparison between the expression level of a marker in an adult stromal cell for use in the invention, and the expression level of the same marker in another cell, such as for example an embryonic stem cell, may preferably be conducted by comparing the two cell types that have been isolated from the same species. Preferably this species is a mammal, and more preferably this species is human. Such comparison may conveniently be conducted using a reverse transcriptase polymerase chain reaction (RT-PCR) experiment.

Cell-surface markers can be identified by any suitable conventional technique, usually based on a positive/negative selection; for example, monoclonal antibodies against cell-surface markers, whose presence/absence in the cells is to be confirmed, can be used; although other techniques can also be used. Thus, in a particular embodiment, monoclonal antibodies against one, two, three, four, five, six, seven of or all of CD9, CD10, CD13, CD29, CD44, CD49A, CD51, CD54, CD55, CD58, CD59, CD90 and CD105 are used in order to confirm the absence of said markers in the selected cells; and monoclonal antibodies against one, two, three, four, of or all of Factor VIII, alpha-actin, desmin, S-100, keratin ,CD11b, CD11c, CD14, CD45, HLAII, CD31, CD34, CD45, STRO-1 and CD133 are used in order to confirm the presence thereof or detectable expression levels of, at least one of and preferably all of, said markers.

In a further embodiment monoclonal antibodies against at least one, two, three or all of CD34; CD45; CD31; CD14 e.g. CD31 and/or CD34 are used in order to confirm the presence or detectable expression levels of said markers. In a further embodiment, monoclonal antibodies against CD34 are used in order to confirm the absence of said marker in the selected cells. In a further embodiment, monoclonal antibodies against STRO-1 is used in order to confirm the absence of said marker in the selected cells. In a further embodiment monoclonal antibodies against at least one, two, three or all of CD29; CD59; CD90; CD105 e.g. CD59 and/or CD90 are used in order to confirm the presence or detectable expression levels of thereof.

Said monoclonal antibodies are known, commercially available or can be obtained by a skilled person in the art by conventional methods.

IFN-γ-inducible IDO activity in the selected cells can be determined by any suitable conventional assay. For example, the selected cells can be stimulated with IFN-γ and assayed for IDO expression; then conventional Western-blot analysis for IDO protein expression can be performed and IDO enzyme activity following IFN-γ stimulation of the selected cells can be measured by tryptophan-to-kynurenine conversion with for example via High Performance Liquid Chromatography (HPLC) analysis and photometric determination of kynurenine concentration in the supernatant as the readout. Since the cells for use in the invention express IDO under certain conditions, any suitable technique which allows the detection of IDO activity following IFN-γ stimulation may be used for selecting the cells for use in the invention. A suitable assay for determining IFN-γ-inducible IDO activity in the selected cells is disclosed in WO2007039150. The amount of IDO produced depends on the number of cells per square centimetre, which is preferably at a level of 5000cells/cm² or more, but not limited to this concentration and the concentration of IFN- γ, which ideally is 3ng/ml or more, but not limited to this concentration. The activity of IDO produced under the described conditions will result in a detectable production of kynurenine in the µM range after 24 hours or more.

### ADMINISTRATION

A composition may be prepared for systemic administration (e.g. rectally, nasally, buccally, vaginally, via an implanted reservoir or via inhalation). A composition may be prepared for local administration. A composition may be administered by the parenteral route. A composition may be administered by the subcutaneous, intracutaneous, intravenous, intramuscular, intra articular, intrasynovial, intrasternal, intrathecal, intralesional, intralymphatic and intracranial routes. The preferred route of administration is by intravenous. Preferred MSCs are ASCs.

Thus, the invention provides ASCs, for use in treating sepsis secondary to pneumonia in a human patient, wherein the MSCs are to be administered by an intravenous route. In one embodiment, the invention provides ASCs for use in treating sepsis secondary to pneumonia in a human patient, wherein the ASCs are administered by an intravenous route, for example by intravenous injection, e.g. by intralymphatic injection to a lymphatic organs such as a peripheral lymphatic organ, including but not limited to the lymph nodes, most preferably an axillary or inguinal lymph node. In each of these embodiments, the MSCs are ASCs, for example eASCs. As used herein, the term "lymphatic system" is to be given its usual meaning in the art and refers to lymphoid tissue, such as a lymphatic organ, connected by a conducting system of lymph vessels and lymph capillaries. The term "lymphatic organ" refers to lymph nodes, most preferably an axillary or inguinal lymph node. The ASCs are not directly administered to lung or by the intratracheal route. In one embodiment, the MSCs used in the invention may be autologous with respect to the subject to be treated. In a further embodiment, the MSCs used in the invention may be allogeneic or xenogeneic with respect to the subject to be treated. Allogenic adipose tissue-derived stromal cells derived from a donor may theoretically be used for the treatment of any patient, irrespective of MHC incompatibility.

In one embodiment, the composition for use in the invention may be administered by injection or implantation of the composition at one or more target sites in the subject to be treated. In a further embodiment, the composition for use in the invention may be inserted into a delivery device which facilitates introduction of the composition into the subject by injection or implantation. In one embodiment the delivery device may comprise a catheter. In a further embodiment, the delivery device may comprise a syringe.

### DOSAGE

The dosage of MSCs and any further therapeutic agent will vary depending on the symptoms, age and body weight of the patient, the nature and severity of the disorder to be treated or prevented, the route of administration, and the form of the further therapeutic agent. The compositions for use in the invention may be administered in a single dose or in divided doses. Appropriate dosages for MSCs and any further therapeutic agent(s) may be determined by known techniques.

Typically said dose is about 10 x 10⁶ cells/kg of subject weight or lower, is about 9 x 10⁶ cells/kg or lower, is about 8 x 10⁶ cells/kg or lower, is about 7 x 10⁶ cells/kg or lower, is about 6 x 10⁶ cells/kg or lower, is about 5 x 10⁶ cells/kg or lower. In an alternative embodiment said dose may be between about 0.25 x 10⁶ cells/kg to about 5 x 10⁶ cells/kg; or more preferably about 1 x 10⁶ cells/kg to about 5 x 10⁶ cells/kg. Accordingly in further alternative embodiments the dose may be about 0.25 x 10⁶ cells/kg, 0.5 x 10⁶ cells/kg, 0.6 x 10⁶ cells/kg, 0.7 x 10⁶ cells/kg; 0.8 x 10⁶ cells/kg; 0.9 x 10⁶ cells/kg; 1.1 x 10⁶ cells/kg; 1.2 x 10⁶ cells/kg; 1.3 x 10⁶ cells/kg; 1.4 x 10⁶ cells/kg; 1.5 x 10⁶ cells/kg; 1.6 x 10⁶ cells/kg; 1.7 x 10⁶ cells/kg; 1.8 x 10⁶ cells/kg; 1.9 x 10⁶ cells/kg or 2 x 10⁶ cells/kg. The dose may, in other embodiments, be between 0.1 and 1 million cells / kg; or between 1 and 2 million cells / kg; or between 2 and 3 million cells / kg; or between 3 and 4 million cells / kg; or between 4 and 5 million cells / kg; or between 5 and 6 million cells / kg; or between 6 and 7 million cells / kg; or between 7 and 8 million cells / kg; or between 8 and 9 million cells / kg; or between 9 and 10 million cells / kg.

In an alternative embodiment the cells may be administered to the patient as a fixed dose, independent of patient weight. Typically said dose is between about 10 million cells and 500 million cells, e.g. said dose is about 10 x 10⁶ cells, 50 x 10⁶ cells, 10 x 10⁷ cells, 50 x 10⁷ cells.

Thus, in one embodiment the invention provides ASCs, for use in treating sepsis secondary to pneumonia, such as severe sepsis secondary to pneumonia, in a human subject wherein the MSCs are administered by an intravenous route, for example by intravenous injection, at a dose of between about 0.25 x 10⁶ cells/kg to about 5 x 10⁶ cells/kg of subject weight. A typical dose is 4 x 10⁶ cells/kg of subject weight. The sepsis may be secondary to bacterial pneumonia (*e.g*. gram-negative pneumonia).

In another embodiment, which can optionally be combined with the above embodiment, the invention provides ASCs, for use in treating sepsis secondary to pneumonia, such as severe sepsis secondary to pneumonia, in a human subject wherein the MSCs are administered by an intravenous route repeatedly, *i.e.* two or more doses are administered, for example at least 3 doses, at least 4, 5, 6, 7, 8, 9, or 10 doses. In one embodiment, two doses are administered. In another embodiment, three doses are administered. In a further embodiment, four doses are administered. The interval between doses can be one day, one week, or one month. Typically, the interval is one day. The MSCs can be administered over a period of no more than 1 week (*e.g*. with daily intervals, such as at days 1 and 3, or days 1, 3 and 5, or days 1, 3, 5 and 7). In one embodiment, two doses of MSCs are administered with one day interval, *i.e.* at day 1 and day 3 (day 1 denotes the first dose of MSCs).

The precise time of administration and amount of any particular agent that will yield the most effective treatment in a given patient will depend upon the activity, pharmacokinetics, and bioavailability of the agent, the physiological condition of the patient (including age, sex, disease type and stage, general physical condition, responsiveness to a given dosage and type of medication), the route of administration, *etc..* The information presented herein may be used to optimize the treatment, e.g., determining the optimum time and/or amount of administration, which will require no more than routine experimentation, such as monitoring the subject and adjusting the dosage and/or timing. While the subject is being treated, the health of the subject may be monitored by measuring one or more of relevant indices at predetermined times during a 24-hour period. Treatment regimens, including dosages, times of administration and formulations, may be optimized according to the results of such monitoring.

Treatment may be initiated with smaller dosages which are less than the optimum dose. Thereafter, the dosage may be increased by small increments until the optimum therapeutic effect is attained.

The combined use of several therapeutic agents may reduce the required dosage for any individual component because the onset and duration of effect of the different components may be complimentary. In such combined therapy, the different active agents may be delivered together or separately, and simultaneously or at different times within the day.

### ADJUVANT THERAPIES

In one embodiment, the pharmaceutical composition for use in the invention may contain or alternatively may be administered in conjunction with one or more (or two or more, or three or more, e.g. 1, 2, 3, 4 or 5) further therapeutic agents.

In some embodiments, the MSCs and the one or more further therapeutic agents may be administered to the subject simultaneously. In other embodiments, the MSCs and the one or more further therapeutic agents may be administered to the subject sequentially. The one or more further therapeutic agents may be administered before or after administration of the MSCs.

Said therapeutic agent may be selected from the following: an analgesic, such as a nonsteroidal anti-inflammatory drug, an opiate agonist or a salicylate; an anti-infective agent, such as an antihelmintic, an antianaerobic, an antibiotic, an aminoglycoside antibiotic, an antifungal antibiotic, a broad-spectrum antibiotic, a cephalosporin antibiotic, a macrolide antibiotic, a β-lactam antibiotic, a penicillin antibiotic, a quinolone antibiotic, a sulfonamide antibiotic, a tetracycline antibiotic, an antimycobacterial, an antituberculosis antimycobacterial, an antiprotozoal, an antimalarial antiprotozoal, an antiviral agent, an anti-retroviral agent, a scabicide, an anti-inflammatory agent, a corticosteroid anti-inflammatory agent, an antipruritics/local anesthetic, a topical anti-infective, an antifungal topical anti-infective, an antiviral topical anti-infective; an electrolytic and renal agent, such as an acidifying agent, an alkalinizing agent, a diuretic, a carbonic anhydrase inhibitor diuretic, a loop diuretic, an osmotic diuretic, a potassium-sparing diuretic, a thiazide diuretic, an electrolyte replacement, and an uricosuric agent; an enzyme, such as a pancreatic enzyme and a thrombolytic enzyme; a gastrointestinal agent, such as an antidiarrheal, an antiemetic, a gastrointestinal anti-inflammatory agent, a salicylate gastrointestinal anti-inflammatory agent, an antacid anti-ulcer agent, a gastric acid-pump inhibitor anti-ulcer agent, a gastric mucosal anti-ulcer agent, a H2-blocker anti-ulcer agent, a cholelitholytic agent, a digestant, an emetic, a laxative and stool softener, and a prokinetic agent; a general anesthetic, such as an inhalation anesthetic, a halogenated inhalation anesthetic, an intravenous anesthetic, a barbiturate intravenous anesthetic, a benzodiazepine intravenous anesthetic, and an opiate agonist intravenous anesthetic; a hormone or hormone modifier, such as an abortifacient, an adrenal agent, a corticosteroid adrenal agent, an androgen, an anti-androgen, an immunobiologic agent, such as an immunoglobulin, an immunosuppressive, a toxoid, and a vaccine; a local anesthetic, such as an amide local anesthetic and an ester local anesthetic; a musculoskeletal agent, such as an anti-gout anti-inflammatory agent, a corticosteroid anti-inflammatory agent, a gold compound anti-inflammatory agent, an immunosuppressive anti-inflammatory agent, a non-steroidal anti-inflammatory drug (NSAID), a salicylate anti-inflammatory agent, a mineral; and a vitamins, such as vitamin A, vitamin B, vitamin C, vitamin D, vitamin E, and vitamin K.

In another embodiment, the further therapeutic agent may be a growth factor or other molecule that affects cell proliferation or activation. In a further embodiment that growth factor may induce final differentiation. In another embodiment, the growth factor may be a variant or fragment of a naturally-occurring growth factor. Methods of producing such variants are well known in the art, and may include, for example, making conservative amino acid changes, or by mutagenesis and assaying the resulting variant for the required functionality.

The invention will now be further illustrated by the following examples. These examples are provided by way of illustration only, and are not intended to be limiting.

### EXAMPLE 1

### Effect of ASC treatment on pneumonia-associated sepsis

**Objective:** To determine the effect of ASCs administered at 6h post infection on the progression and dissemination of bacteria, and on the levels of pro-inflammatory cytokines in the lung.

### Mice:

- Eight to twelve-week old, specific pathogen-free female C57BL/6 mice
- Mice were randomized and housed for 7 days prior to the experiment for acclimatization
- Animals in experiment were observed twice a day. If extremely ill, more frequent (6x) observation was required and essential in accordance with the Animal Ethical Committee

### Pneumonia:

Gram-negative pneumonia was induced by intranasal instillation of live *Klebsiella pneumoniae* serotype2 (1 x 10⁴ CFU; ATCC 43816; American Type Culture Collection). Bacteria were cultured in Tryptic Soy Broth (TSB) medium and blood agar (BA) plates.

### Day -1: Start culture

One bead of -80 °C *Klebsiella pneumoniae* stock were placed in 50 ml TSB medium and grown overnight (16 hours) at 37 °C.

### Day 0: Preparing inoculum

1 ml of the culture (day -1) was resuspended into 100 ml (warm) TSB medium and grown until OD=1.0 (± 2.5-3 hours). 10 ml culture was washed with cold sterile 0.9% NaCl (Baxter) at centrifuge at 4000 rpm for 10min at 4°C. Supernatant was removed and washed 2 times more as described above. Pellet was resuspended in 10 ml NaCl, the bacterial concentration was approximately 0.4 x 10⁹ CFU/ml. This was diluted 2000 times to about 20 x 10⁴ CFU/ml. The concentration of the inoculum was checked by plating 50 µl of 10²-10⁴ dilutions of the inoculum on three BA plates. The CFU were counted after overnight culture at 37 °C (Day 1). Mice were inoculated:

### Inoculum: 50 µl/mouse → 1 x 10⁴ CFU

### Stem cells:

ASCs (1 x 10⁶ expanded human adipose tissue derived stem cells) or placebo (Ringer's lactate) were administered intravenously at 6 hours after intranasal inoculation of the mice.

### Study design

### T= 0 hours

- Isofluran anesthetic
- All mice: intranasal instillation of live *Klebsiella pneumoniae* serotype 2 (50 µl, 1 x 10⁴ CFU)

### T= 6 hours

- Group I: (n=8) 1 x intravenous (IV) injected with 200 µl placebo (Ringer's lactate)
- Group II: (n = 8) 1 x IV injected with 1 x 10⁶ ASCs (in 200 µl Ringer's lactate)

### T= 48 hours: Salification of mice

### Study endpoints

• *Bacterial loads in lungs, spleen, liver and blood (semi-quantitative cultures)*
• *Inflammation: cytokine (TNF-α, IL-1β IL-6) and chemokine (MIP-2) in lung homogenates*

### Results:

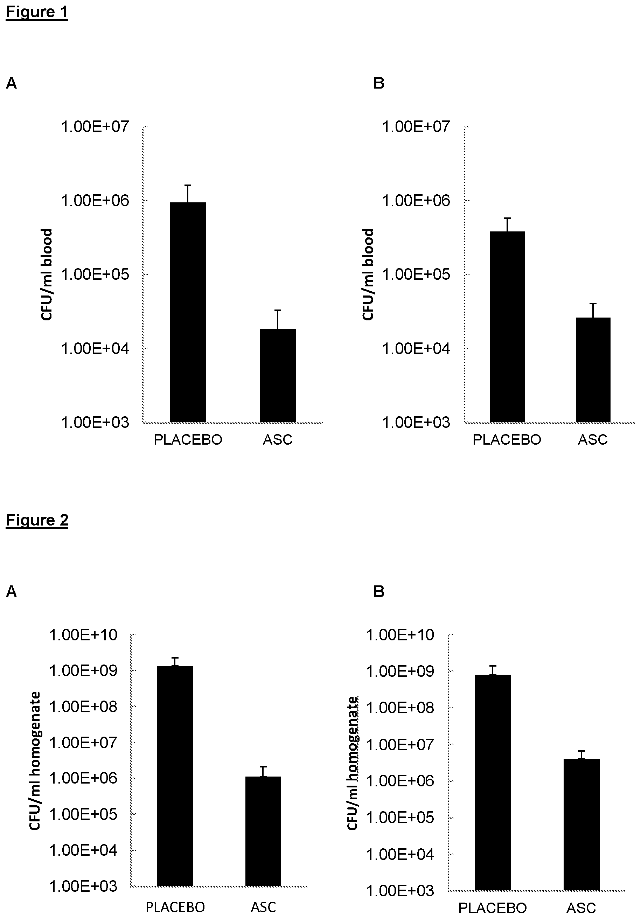

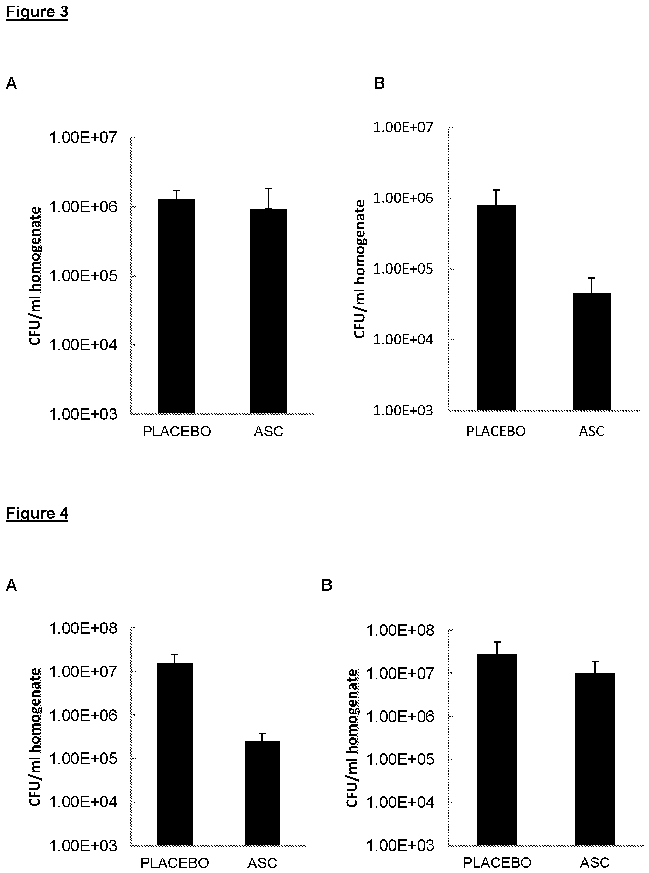

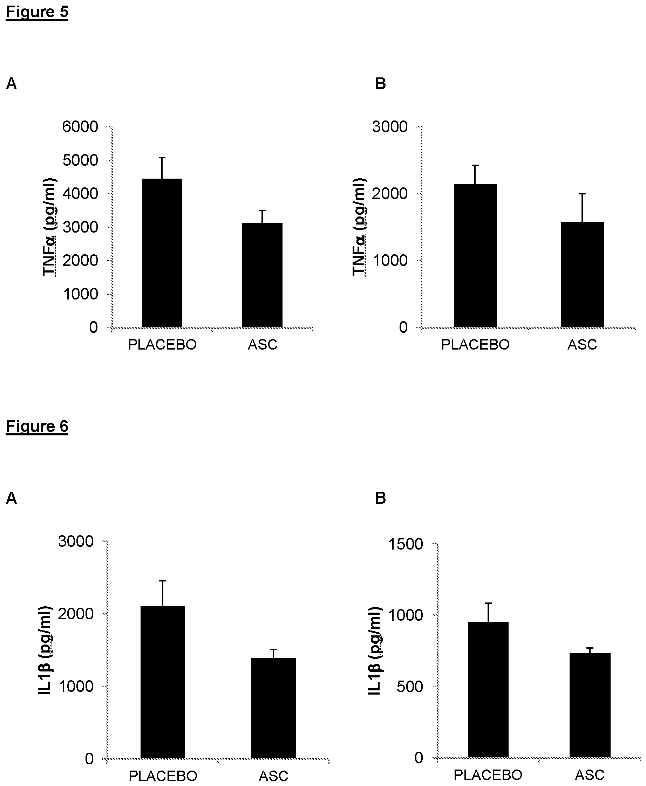

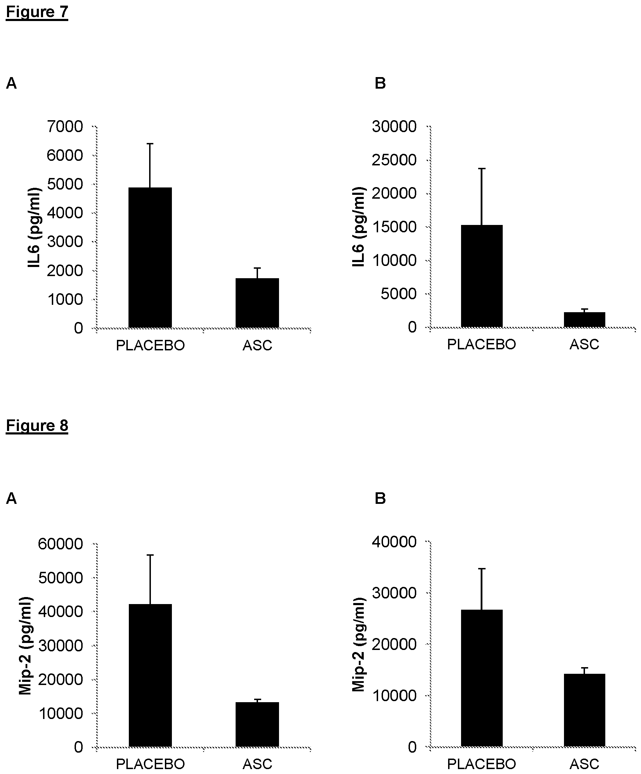

Figures 1-8 show the results from two independent experiments (labelled A and B, respectively). As shown in Figures 1-4, compared to placebo, treatment with ASCs after 6h of infection reduced bacterial load in blood (Figure 1) and lungs (Figure 2) and also reduced bacterial loads in liver (Figure 3) or spleen (Figure 4). Moreover, as shown in Figures 5-8, levels of pro-inflammatory cytokines and chemokines were reduced in lung homogenates after treatment with ASCs, compared to placebo. These results demonstrate that treatment with ASCs reduces the severity of pneumonia-associated sepsis. Bacterial loads and pro-inflammatory cytokine/chemokine levels were reduced in various issues, including target organs relevant to pneumonia such as blood and lungs. MSCs, such as ASCs, are therefore surprisingly useful in the treatment of pneumonia-associated sepsis.

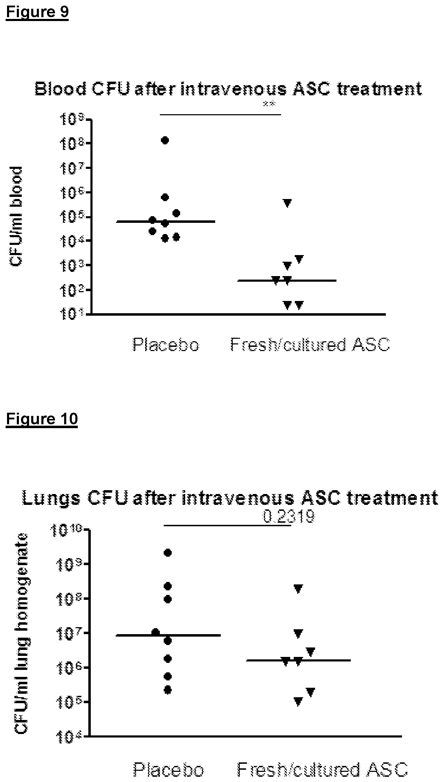

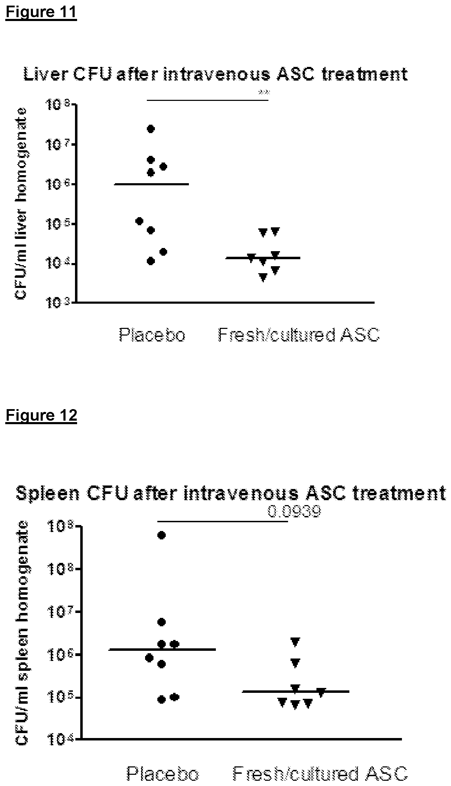

Results from a further study are shown in Figures 9-12, again demonstrating that intravenous administration of ASCs in pneumonia-associated sepsis reduces the bacterial load in various organs such as blood.

### EXAMPLE 2

A clinical trial is conducted in severe pneumonia patients with severe sepsis or septic shock. Patients are enrolled within 24 hours of diagnosis of sepsis.

Approximately half of patients enrolled in the study receive treatment with a medicinal product consisting of a suspension of donor-derived (allogeneic) expanded adipose stromal cells (eASCs) in Ringer's lactate solution. The other half receive a placebo consisting of a suspension of Ringer's lactate solution. Patients in the treatment group receive a single dose of 4 million cells/kg patient body weight of the medicinal product. Patients in the placebo group receive a single dose of the placebo. In both cases administration is by means of intravenous infusion.

The medicinal product is a cell suspension in sterile buffer solution containing adipose-derived stromal cells (eASCs) of allogeneic origin in disposable vials, obtained through lipoaspiration from healthy individuals and expanded in vitro. The medicinal product is supplied as a sterile, clear, colourless suspension for intralesional administration, provided in 6 mL vials (suspension of 10 million eASCs per mL of Dulbecco modification Eagle's medium [DMEM] with human serum albumin). The medicinal product will be administered after suspension in Ringer's lactate solution at an infusion rate of 4ml/min.

The study placebo is a Ringer's lactate solution for intralesional administration at an infusion rate of 4ml/min. The corresponding placebo volume (Ringer's lactate solution) will be administered to each subject from the placebo groups. Placebo volume will be calculated according to the subject's weight.

### Medicinal product preparation

The allogeneic eASCs medicinal product consists of a cellular suspension of living adult stromal cells extracted from the subdermal adipose tissue of healthy donors. Subdermal adipose tissue is liposuctioned from the healthy donor and transported to a GMP manufacturing facility. The donation, procurement, and testing are carried out according to the requirements of Directive 2004/23/EC and therefore under Directives 2006/17/EC and 2006/83/EC. ASCs are isolated by digesting the adipose tissue with type I collagenase, followed by centrifugation. The cell pellet obtained is resuspended and lysed in erythrocyte lysis solution and centrifuged. The stromal vascular fraction, resulting from the cell pellet, is placed in cell culture containers in culture medium and antibiotics, and incubated at 37 °C and 5 % CO2 and in a humidified atmosphere. At 24-48 h post-plating, the culture medium is removed to eliminate the non-attached cell fraction. ASCs adhered to the plastic culture plates are expanded under in vitro conditions. Every 3-4 days, the culture medium is changed after reaching 90-95 % confluence and the cells are detached with trypsin/EDTA, collected, centrifuged, and expanded without antibiotics to the required duplication. They are then harvested and cryopreserved until use. Before the appointed administration date, sufficient cryopreserved vials are thawed to provide the required dose for administration. ASCs are recovered from their cryopreserved state by plating and culturing (to confirm viability). On the day when the vials are filled and packaged, the cultures were washed with phosphate buffer solution, and trypsin/EDTA. The ASCs are immediately resuspended in the selected excipients (Dulbecco modification Eagle medium and human albumin serum) to formulate the drug product.

The eASCs are characterized in terms of identity (phenotypic profile), purity, potency, morphology, viability, and cell growth kinetics according to the Guideline on Cell- Based Medicinal Products (EMEA/CHMP/410869/2006) and the Reflection Paper on Stem Cells (EMA/CAT/ 571134/2009).

## Claims

1. A composition comprising adipose tissue-derived stromal cells (ASCs) for use in treating sepsis in a human subject, wherein the sepsis is secondary to an inflammatory lung condition and wherein the composition is administered by an intravenous route.

2. The composition for use according to claim 1, wherein the sepsis is severe sepsis.

3. The composition for use according to claim 1 or claim 2, wherein the inflammatory lung condition is pneumonia.

4. The composition for use according to claim 3, wherein the pneumonia is caused by bacteria.

5. The composition for use according to any preceding claim, wherein the cells are expanded adipose tissue-derived stromal cells.

6. The composition for use according to any preceding claim, wherein the ASCs are allogeneic.

7. The composition for use according to any preceding claim, wherein the composition comprises between about 0.25 x 10⁶ cells/kg to about 5 x 10⁶ cells/kg of subject weight.

8. The composition for use according to any preceding claim, wherein the ASCs are administered to the subject repeatedly, for example at day 1 and day 3.

9. The composition for use according to any preceding claim, wherein at least about 50% of the ASCs express one or more of the markers CD9, CD10, CD13, CD29, CD44, CD49A, CD51, CD54, CD55, CD58, CD59, CD90 and CD105.

10. The composition for use according to any preceding claim, wherein at least about 50% of the ASCs do not express the markers Factor VIII, alpha-actin, desmin, S-100, keratin, CD11b, CD11c, CD14, CD45, HLAII, CD31, CD34, CD45, STRO-1 and CD133.

11. The composition for use according to any preceding claim, wherein the ASCs are administered in a pharmaceutically acceptable carrier and/or a diluent.

12. The composition for use according to any preceding claim, wherein the ASCs are administered in conjunction with one or more further therapeutic agents.

## Patentansprüche

1. Zusammensetzung umfassend Stroma-Zellen aus Fettgewebe (ASCs) zur Verwendung in der Behandlung von Sepsis in einem menschlichen Subjekt, wobei die Sepsis sekundär zu einer entzündlichen Lungenerkrankung ist und wobei die Zusammensetzung auf intravenösem Weg verabreicht wird.

2. Zusammensetzung zur Verwendung nach Anspruch 1, wobei die Sepsis schwere Sepsis ist.

3. Zusammensetzung zur Verwendung nach Anspruch 1 oder 2, wobei die entzündliche Lungenerkrankung Pneumonie ist.

4. Zusammensetzung zur Verwendung nach Anspruch 3, wobei die Pneumonie von Bakterien verursacht wird.

5. Zusammensetzung zur Verwendung nach einem der vorhergehenden Ansprüche, wobei die Zellen expandierte Stroma-Zellen aus Fettgewebe sind.

6. Zusammensetzung zur Verwendung nach einem der vorhergehenden Ansprüche, wobei die ASCs allogen sind.

7. Zusammensetzung zur Verwendung nach einem der vorhergehenden Ansprüche, wobei die Zusammensetzung zwischen etwa 0,25 x 10⁶ Zellen/kg bis etwa 5 x 10⁶ Zellen/kg Gewicht des Subjekts umfasst.

8. Zusammensetzung zur Verwendung nach einem der vorhergehenden Ansprüche, wobei die ASCs dem Subjekt wiederholt verabreicht werden, zum Beispiel an Tag 1 und Tag 3.

9. Zusammensetzung zur Verwendung nach einem der vorhergehenden Ansprüche, wobei mindestens etwa 50% der ASCs einen oder mehrere der Marker CD9, CD 10, CD13, CD29, CD44, CD49A, CD51, CD54, CD55, CD58, CD59, CD90 und CD105 exprimieren.

10. Zusammensetzung zur Verwendung nach einem der vorhergehenden Ansprüche, wobei mindestens etwa 50% der ASCs nicht die Marker Faktor VIII, Alpha-Aktin, Desmin, S-100, Keratin, CD11b, CD11c, CD14, CD45, HLAII, CD31, CD34, CD45, STRO-1 und CD133 exprimieren.

11. Zusammensetzung zur Verwendung nach einem der vorhergehenden Ansprüche, wobei die ASCs in einem pharmazeutisch akzeptablen Träger und/oder Verdünnungsmittel verabreicht werden.

12. Zusammensetzung zur Verwendung nach einem der vorhergehenden Ansprüche, wobei die ASCs in Verbindung mit einem oder mehreren weiteren therapeutischen Wirkstoffen verabreicht werden.

## Revendications

1. Composition comprenant des cellules stromales dérivées de tissu adipeux (ASC) pour utilisation dans le traitement de la septicémie chez un sujet humain, sachant que la septicémie est secondaire à un état pulmonaire inflammatoire et sachant que la composition est administrée par voie intraveineuse.

2. La composition pour utilisation selon la revendication 1, sachant que la septicémie est une septicémie grave.

3. La composition pour utilisation selon la revendication 1 ou la revendication 2, sachant que l'état pulmonaire inflammatoire est une pneumonie.

4. La composition pour utilisation selon la revendication 3, sachant que la pneumonie est causée par des bactéries.

5. La composition pour utilisation selon une quelconque revendication précédente, sachant que les cellules sont des cellules stromales dérivées de tissu adipeux expansées.

6. La composition pour utilisation selon une quelconque revendication précédente, sachant que les ASC sont allogéniques.

7. La composition pour utilisation selon une quelconque revendication précédente, sachant que la composition comprend entre environ 0,25 x 10⁶ cellules/kg et environ 5 x 10⁶ cellules/kg de poids de sujet.

8. La composition pour utilisation selon une quelconque revendication précédente, sachant que les ASC sont administrées au sujet de manière répétée, par exemple au jour 1 et au jour 3.

9. La composition pour utilisation selon une quelconque revendication précédente, sachant qu'au moins environ 50 % des ASC expriment un ou plusieurs des marqueurs CD9, CD10, CD13, CD29, CD44, CD49A, CD51, CD54, CD55, CD58, CD59, CD90 et CD105.

10. La composition pour utilisation selon une quelconque revendication précédente, sachant qu'au moins environ 50 % des ASC n'expriment pas les marqueurs Facteur VIII, alpha-actine, desmine, S-100, kératine, CD11b, CD11c, CD14, CD45, HLAII, CD31, CD34, CD45, STRO-1 et CD133.

11. La composition pour utilisation selon une quelconque revendication précédente, sachant que les ASC sont administrées dans un support et/ou diluant pharmaceutiquement acceptable.

12. La composition pour utilisation selon une quelconque revendication précédente, sachant que les ASC sont administrées en conjonction avec un ou plusieurs agents thérapeutiques supplémentaires.
